# FASCICULE DE BREVET EUROPEEN

(11) **EP 0 666 104 B1**
(45) Date de publication et mention de la délivrance du brevet: **11.11.1998**
(21) Numéro de dépôt: 95400164.0
(22) Date de dépôt: 25.01.1995
(51) Int. Cl.: B01J 12/00, B01J 19/24, C07C 2/76, F23C 3/00

(54) **Dispositif pour la mise en oeuvre de réactions chimiques nécessitant au moins au démarrage un apport de calories**
Vorrichtung zur Durchführung von chemischen Reaktionen welche, mindestens während des Startens, eine Zufuhr von Kalorien nötig haben
Apparatus for carrying out chemical reactions which, at least during start-up, require a supply of calories

(30) Priorité: 02.02.1994 FR 9401243
(43) Date de publication de la demande: 09.08.1995
(73) Titulaire: INSTITUT FRANCAIS DU PETROLE, 92502 Rueil-Malmaison (FR); GAZ DE FRANCE, 75017 Paris (FR)
(72) Inventeur: Busson, Christian, F-69260 Charbonnierre (FR); Delhomme, Henri, F-69110 Sainte Foy Les Lyon (FR); Gollion, Laure, F-75019 Paris (FR); Cassagne, Jean Pierre, F-94120 Fontenay Sous Bois (FR)
(74) Mandataire: Andreeff, François

(56) Documents cités:
- EP-A- 0 539 270
- FR-A- 2 403 518
- US-A- 2 163 599
- US-A- 5 160 501
- REVUE GENERALE DE THERMIQUE, vol.29, no.346, Octobre 1990, PARIS FR pages 531 - 538 L.SCRIVE & J.P.CASSAGNE 'Cerajet: gas-fired radiant tube for indirect heating of high-temperature industrial furnaces'

## Description

La présente invention concerne un dispositif pour la mise en oeuvre de réactions chimiques nécessitant au moins au démarrage un apport de calories. Ce dispositif comprend habituellement une série d'éléments permettant au moins dans une première zone un apport de chaleur nécessaire au démarrage de la réaction. Ce dispositif est utilisable pour des réactions globalement exothermiques, mais nécessitant l'apport de calories au démarrage telle que par exemple les réactions d'hydrogénation catalytiques. Ce dispositif est plus particulièrement applicable à la mise en oeuvre de toute réaction endothermique mais plus particulièrement pour la mise en oeuvre des réactions de vapocraquage, de pyrolyse, de déshydrogénation catalytique et de vaporéformage catalytique d'hydrocarbures ou de coupes d'hydrocarbures dans lesquelles la température de réaction est le plus souvent supérieure à environ 350 °C et où l'un des problèmes à résoudre est de limiter les réactions secondaires conduisant à la formation de goudron et/ou de coke.

De très nombreux documents décrivent des procédés et des dispositifs pour la mise en oeuvre de ces procédés. On peut en particulier citer le brevet US-A-4 780 196 et le brevet US-A-4 973 777 au nom de l'Institut Français du Pétrole qui décrivent respectivement un procédé de pyrolyse thermique en présence de vapeur d'eau, dit procédé de vapocraquage, et un procédé de conversion thermique du méthane, mis en oeuvre dans un réacteur multicanaux en matière céramique. Le procédé de vapocraquage donne de bons rendements en éthylène et en propylène et le couplage thermique déshydrogénant du méthane fourni de bons rendements en acétylène, éthylène et composés aromatiques. Cependant la conception du réacteur est délicate, les céramiques employées pour sa réalisation sont des céramiques relativement chères et il est difficile de maintenir une température constante tout au long de la zone réactionnelle, ce qui pénalise le procédé. Par ailleurs la conception même du réacteur implique un contrôle de température tout au long de la zone de réaction très difficile dans le cas du vapocraquage et de l'utilisation comme moyen de chauffage d'un fluide de chauffage circulant dans certains canaux. Dans le cas du chauffage électrique pour la conversion thermique du méthane la durée de vie des résistances électriques est d'autant plus limitée que la température que l'on souhaite maintenir dans la zone réactionnelle proprement dite est plus élevée. Par ailleurs la conception du réacteur est également très délicate et le contrôle des sections transversales de chauffage nécessite des systèmes complexes tels que des transformateurs asservis et des modulateurs à thyristors.

Les études thermodynamiques et cinétiques de ces réactions et en particulier des réactions de pyrolyse d'hydrocarbures et de celles concernant la conversion thermique du méthane conduisent donc, afin d'augmenter la sélectivité de la réaction vers la production des produits souhaités, (oléfines ou du mélange oléfines, acétyléniques et aromatiques) à intervenir sur les paramètres suivants :
- augmentation rapide de la température de la charge jusqu'à la température optimale de pyrolyse pour une charge donnée, et maintien de cette température la plus constante possible dans la zone réactionnelle,
- diminution du temps de séjour de la charge dans la partie réactionnelle,
- diminution de la pression partielle de la charge hydrocarbonée.

Il est donc particulièrement important de minimiser le temps de contact entre les produits de la réaction et les parois chaudes du réacteur.

Sur le plan de la technologie, ces impératifs ont rapidement conduit à un schéma général de procédé consistant en :
a) un préchauffage de la charge (éventuellement diluée par de la vapeur d'eau ou de l'hydrogène, de l'azote ou par un autre gaz en fonction de la réaction à effectuer),
b) un chauffage à haute température de cette charge ou du mélange charge-vapeur d'eau ou encore du mélange charge-gaz de dilution, dans des fours tubulaires afin de limiter le temps de séjour des hydrocarbures au cours de cette phase de pyrolyse.

L'évolution des fours de pyrolyse thermique et notamment de vapocraquage a essentiellement été axée vers l'obtention de temps de séjour plus réduits et de diminution de la perte de charge, ce qui a conduit les constructeurs à réduire la longueur des réacteurs tubulaires, donc à augmenter la densité de flux thermique.

L'accroissement de ce dernier facteur peut être essentiellement obtenu en augmentant la température de peau des réacteurs tubulaires et/ou en diminuant le diamètre des tubes (ce qui permet d'augmenter le rapport s/v, s étant la surface d'échange et v le volume réactionnel).

On a également proposé plusieurs modèles de four de pyrolyse, tendant tous à augmenter la densité de flux thermique vers le début du tube de pyrolyse et à la réduire par la suite, soit en utilisant des réacteurs tubulaires de diamètre croissant, soit en rassemblant au moins deux tubes de pyrolyse en un seul après une certaine longueur de zone réactionnelle (voir par exemple l'article de F. WALL et al publié dans Chemical Engineering Progress, décembre 1983, pages 50 à 55) ; on a également décrit des fours tubulaires non cylindriques, tendant à augmenter le rapport s/v ; c'est ainsi que le brevet US-A-3 572 999 décrit l'utilisation de tubes de section ovale et que le brevet US-A-3 964 873 décrit celle de tubes dont la section est en forme d'haltère.

La technologie des réacteurs de pyrolyse thermique et notamment de vapocraquage a ainsi évolué, depuis l'utilisation de tubes horizontaux de 100 mètres (m) de long environ et de diamètres intérieurs de l'ordre de 90 à 140 millimètres (mm), jusqu'à la technique classique de tubes suspendus verticalement de 40 m de longueur environ et de diamètre de l'ordre de 60 mm fonctionnant avec des temps de résidence de l'ordre de 0,3 à 0,4 seconde (s), et enfin la technique dite milliseconde proposée par PULLMAN-KELLOG (brevet US-A-3 671 198) qui utilise des tubes de 10 m environ de longueur, verticaux et rectilignes, de diamètre intérieur de 25 à 35 mm, ces tubes étant portés à des températures de l'ordre de 1 100 °C (température le plus souvent très voisine de celle de la limite d'utilisation du métal). Le temps de résidence des charges est, dans ce type de four, de l'ordre de 0,07 s ; la perte de charge observée est de l'ordre de 0,9 à 1,8 bar (1 bar est égal à 0,1 mégapascal), et le calcul du rapport de la surface d'échange s au volume réactionnel v conduit à des valeurs de l'ordre de 120 m⁻¹.

L'un des problèmes les plus importants que l'on rencontre dans la mise en oeuvre de la pyrolyse thermique et en particulier du vapocraquage d'hydrocarbures est lié à la formation du coke sur les parois des réacteurs. Cette formation est due en grande partie à des réactions secondaires telles que la formation d'hydrocarbures aromatiques polycycliques condensés. Ces réactions sont largement favorisées par le niveau thermique : au dessus de 900 °C une augmentation de la température des parois des réacteurs de 50 °C conduit au doublement de la vitesse de dépôt de coke sur ces parois. Ce phénomène de cokage conduit obligatoirement à des arrêts de runité pour son décokage quel que soit le procédé. Ce phénomène est d'autant plus gênant que l'espace entre parois est réduit.

On a également proposé dans l'art antérieur, en vue de palier aux inconvénients cités ci-devant, notamment dans les brevets EP-B-323 287 et US-A-5 160 501 au nom de l'Institut Français du Pétrole un procédé de conversion thermique du méthane en hydrocarbures de poids moléculaires plus élevés comportant des moyens de chauffage électrique avec transfert de chaleur au mélange gazeux, contenant le méthane à convertir, à travers les parois étanches ou non étanches de gaines en matière céramique qui isolent lesdits moyens de chauffage du mélange gazeux contenant le méthane. Dans ce procédé, la zone de chauffage est chauffée par apport d'énergie électrique à l'aide de résistances électriques et la chaleur dégagée par effet Joule dans ces résistances est transmise, principalement par radiation, aux gaines en matériau céramique disposées autour des résistances de façon non jointive. Les charges gazeuses, qui circulent sensiblement perpendiculairement à l'axe des gaines chauffées, sont chauffées essentiellement par convection. L'un des principaux inconvénients de ces réalisations réside dans la difficulté d'obtenir une température élevée à l'extérieur des gaines sans être obligé de maintenir les résistances à une température proche du maximum que peut supporter le matériau dont elles sont constituées, ce qui implique une durée de vie relativement courte pour ces résistances et donc un arrêt fréquent de l'unité industrielle en vue de leur changement. Il est aussi très important d'éviter au maximum l'apparition de points chauds sur les surfaces d'échange. Par ailleurs dans les dispositifs décrits dans ces brevets, les étanchéités sont effectuées à chaud, de façon dynamique, par l'utilisation d'une injection de gaz dans les gaines des résistances, ce qui est un inconvénient important lorsqu'un incident se produit sur une seule gaine puisqu'il devient alors nécessaire d'interrompre le fonctionnement de l'installation toute entière. De plus, si dans ces réalisations les gaines peuvent former des éléments chauffants lorsque les résistances sont alimentées en courant électrique ou des éléments passifs lorsqu'elles ne sont pas alimentées en courant électrique, elles ne peuvent jamais être utilisées comme élément de refroidissement indirect des produits circulant dans le four. Enfin la température sur toute la longueur du tube enveloppe n'est pas très homogène.

De plus, le brevet US-A-2 163 599 décrit un échangeur de chaleur comportant des double ou triple tubes concentriques contenant dans une première enveloppe annulaire, un fluide liquide d'échange de chaleur tel que de l'eau, de la saumure ou de l'huile qui circule. Cette enveloppe est entourée d'une autre enveloppe annulaire scellée, dans laquelle est stockée, en tant que liquide tampon, du mercure, du plomb, des alliages fusibles divers, du diphényl ou des sels fondus. Des liens externes sont fixés sur cette dernière enveloppe pour faciliter l'échange thermique avec la paroi externe du tube. Ce type d'échangeur à tubes est incompatible avec des températures de réaction pouvant atteindre 1 500 °C et avec un système de modulation, sans inertie, du niveau de température dans un réacteur.

L'arrière plan technologique est illustré enfin par les brevets FR-A-2 403 518, EP-A-0 539 270 et US-A-5 160 501 et la publication dans la Revue Générale de Thermique vol. 29, n° 346, Oct. 90, Paris France, pages 531 à 538.

Un des objets de l'invention est de remédier aux inconvénients décrits ci-devant. Les objectifs que l'on se propose d'atteindre et qui répondent aux problèmes soulevés par l'art antérieur sont essentiellement les suivants :
- être plus homogène en température sur tout le tube enveloppe des moyens d'échange de chaleur, ce qui doit permettre d'obtenir de meilleurs rendements chimiques.
- améliorer les échanges thermiques entre le ou les réactifs, et les surfaces chaudes en contact avec ce mélange.
- augmenter la fiabilité du dispositif et sa facilité de construction et de démontage pour le décokage du réacteur et son entretien. En particulier le dispositif sera capable de continuer à fonctionner même en cas d'incident sur un ou sur quelques moyens d'échange de chaleur.
- maîtriser au mieux les profils de température réactionnelle, ce qui permet d'augmenter les rendements en produits recherchés, comme par exemple en éthylène et propylène dans le cas du vapocraquage thermique d'hydrocarbures, par rapport aux procédés existants.
- disposer d'un appareillage assurant à la fois la souplesse et la flexibilité de la production et permettant des modes de pilotage par exemple par marche/arrêt à des cadences variables ou à haute fréquence tout en conservant une productivité en produits recherchés la plus élevée possible.
- disposer d'un appareillage dans lequel les étanchéités sont effectuées à froid au niveau des parois du réacteur. Ceci permet de s'affranchir d'une séparation supplémentaire des produits en aval du réacteur dans le cas où le gaz d'étanchéité est de nature différente des produits sortant du réacteur et d'avoir un effluent gazeux à traiter plus faible.
- disposer d'un appareillage dont la géométrie de la zone réactionnelle est variable, ce qui permet pour un réacteur à géométrie fixée de pouvoir fonctionner avec des charges très différentes les unes des autres.
- disposer d'un appareillage pouvant fonctionner, suivant les cas, avec une zone de trempe directe éventuellement couplée avec une zone de trempe indirecte ou uniquement avec une zone de trempe indirecte.
- disposer d'un appareillage dans lequel chaque moyen d'échange de chaleur peut fonctionner soit comme élément de chauffe, soit comme élément de refroidissement, soit encore comme élément passif.

La présente invention propose un dispositif pour la mise en oeuvre de divers procédés et en particulier de ceux cités ci-devant apportant des améliorations notables par rapport aux réalisations selon l'art antérieur telles que par exemple une mise en oeuvre plus facile, plus souple et mieux contrôlée. La souplesse d'utilisation est liée à l'emploi d'éléments d'échange de chaleur qui peuvent être totalement indépendants les uns des autres et qui peuvent à volonté être des éléments chauffants, des éléments de refroidissement ou des éléments passifs.

Plus particulièrement, l'invention concerne un dispositif, pour la réalisation de réactions nécessitant au moins au démarrage un apport de calories et plus particulièrement pour la réalisation de réactions endothermiques. Dans un but de simplification la suite de la description de la présente invention sera faite en liaison avec l'utilisation du dispositif de l'invention dans le cadre de sa mise en oeuvre pour la réalisation de réactions endothermiques et plus particulièrement dans le cadre de sa mise en oeuvre pour la pyrolyse du méthane ou pour la pyrolyse thermique d'hydrocarbures ayant au moins 2 atomes de carbone dans leur molécule. Cependant cette description ne doit pas être considérée comme limitative dans l'emploi du dispositif selon l'invention.

Le dispositif selon la présente invention comprend un réacteur (1) de forme allongée selon un axe, de préférence à section carrée ou rectangulaire, comportant à une première extrémité au moins un moyen d'alimentation en au moins un réactif, à l'extrémité opposée au moins un moyen d'évacuation des effluents produits, ledit réacteur comprenant dans une première zone (côté première extrémité) une pluralité de moyens d'échange de de chaleur (3), lesdits moyens d'échange de chaleur (3), sensiblement parallèles entre eux, étant disposés en nappes sensiblement parallèles, perpendiculaires à l'axe du réacteur de façon à définir entre lesdits moyens et/ou les nappes formées par ces moyens, des espaces ou passages pour la circulation du ou des réactifs et/ou des effluents, lesdits moyens d'échange de chaleur (3) étant adaptés à échanger de la chaleur dans lesdits passages par sections transversales successives, indépendantes et sensiblement perpendiculaires à l'axe du réacteur. Certains au moins des moyens d'échange de chaleur (3) comprennent des éléments tubulaires d'échange de chaleur, chacun comportant au moins une enveloppe connectée à un brûleur à gaz pour fournir un gaz de combustion ou un mélange de gaz de combustion en tant que gaz ou mélange de gaz d'échange de chaleur, et au moins un moyen d'évacuation à l'extérieur du réacteur dudit gaz ou mélange de gaz ayant échangé de la chaleur avec le ou les réactifs et/ou effluents, lesdits brûleurs à gaz étant raccordés à des moyens d'alimentation en gaz combustible et en gaz comburant et des moyens d'asservissement des brûleurs et de modulation de la quantité de gaz fourni à ces brûleurs sont interposés entre lesdits moyens d'alimentation et lesdits brûleurs, pour que les moyens d'échange de chaleur soient alimentés soit en produit de combustion issus des brûleurs, soit en gaz comburant exclusivement.

Dans le cadre de la présente invention, le tube comprend au moins une enveloppe ayant une section droite de forme quelconque par exemple polygonale, ovale ou sensiblement circulaire. Dans le cas d'utilisation de tube dont la section droite de l'enveloppe a une forme polygonale on emploiera avantageusement des tubes dont la section droite de l'enveloppe est de forme triangulaire, carrée, rectangulaire, pentagonale, hexagonale, heptagonale ou octogonale. Le plus souvent cette section droite sera carrée, rectangulaire, ovale ou sensiblement circulaire. Le plus souvent cette section droite sera sensiblement circulaire. Il n'est pas nécessaire que la section soit constante sur toute la longueur du tube, ni dans sa forme, ni dans sa surface, cependant cette section est le plus souvent constante dans sa forme et de préférence également dans sa surface.

La forme des tubes n'est pas très critique et on peut utiliser des tubes comprenant une enveloppe de forme allongée le long d'un axe, des tubes en épingle ou en U, ou des tubes en forme de W. On préfère habituellement employer des tubes comprenant une enveloppe de forme allongée le long d'un axe.

Tout au long du réacteur on peut employer des tubes de formes différentes. Par exemple des tubes en forme de U ou de W au début du réacteur à proximité de l'entrée du ou des réactifs et ensuite des tubes comprenant une enveloppe de forme allongée le long d'un axe. Des tubes en forme de U ou de W peuvent aussi être employés à proximité de l'extrémité opposée à celle par laquelle entre le ou les réactifs.

Dans une forme préférée de réalisation de l'invention, au moins certains des moyens d'échange de chaleur (3) sont formés par un tube en doigt de gant, formé d'une enveloppe extérieure fermée à l'une de ses extrémités et d'une enveloppe intérieure, sensiblement coaxiale à ladite enveloppe extérieure, ouverte à ses deux extrémités et alimentée en gaz ou mélange de gaz d'échange de chaleur par l'une de ses extrémités, ledit gaz ou mélange de gaz d'échange de chaleur étant évacué par l'autre extrémité dans l'espace libre (on dit souvent espace annulaire sans que cela ne limite la forme de cet espace à celle d'un cercle) entre les deux enveloppes.

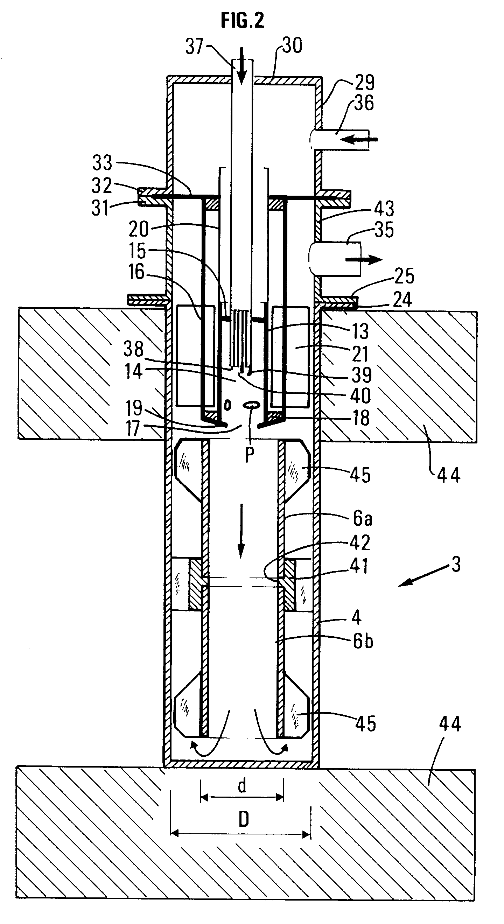

Il est habituellement préférable pour avoir la meilleure homogénéité de température tout au long de l'extérieur de l'enveloppe extérieure du tube en doigt de gant et une bonne circulation des gaz dans l'espace annulaire entre les deux enveloppes que le rapport de l'aire interne (Sₑ) de la section droite du tube formant l'enveloppe extérieure du tube en doigt de gant à l'aire externe (sᵢ) de la section droite (les aires sont calculées à partir des diamètres D et d des tubes de section circulaire selon la réalisation schématisée sur la figure 2) du tube formant l'enveloppe intérieure du tube en doigt de gant soit d'environ 1,4 : 1 à environ 25 : 1 et de préférence d'environ 2 : 1 à environ 9 : 1. Dans ces conditions de rapport d'aires on peut assurer une homogénéité de température telle que sa variation maximale le long du tube externe soit d'au plus 30 °C.

Dans une forme préférée de réalisation, le dispositif de la présente invention comprendra un réacteur ayant dans une deuxième zone (8) (côté extrémité opposée) contiguë à la première zone, des moyens de refroidissement (9) des effluents reliés à au moins un moyen d'alimentation en fluide de refroidissement. Il est habituellement préférable que ce réacteur comporte des moyens d'asservissement et de modulation de l'échange de chaleur reliés aux moyens d'échange de chaleur (3).

La souplesse d'utilisation est liée à l'emploi d'éléments d'échange de chaleur qui peuvent être totalement indépendants les uns des autres et qui permettent d'obtenir une zone réactionnelle de longueur variable sans être limité au départ par la géométrie du réacteur comme cela sera montré ci-après par les exemples d'applications. Les éléments d'échange de chaleur peuvent à volonté être des éléments chauffants, des éléments de refroidissement ou des éléments passifs et donc permettent une variation très aisée de la longueur des différentes zones et des températures dans ces zones.

Les moyens d'échange de chaleur sont habituellement sensiblement perpendiculaires au sens de l'écoulement du ou des produits dans ledit réacteur et forment en projection transversale un faisceau à pas triangulaire, carré ou rectangulaire. Ces moyens peuvent être regroupés par sections successives transversales sensiblement perpendiculaires à la direction (à l'axe) de la zone de réaction, indépendantes entre elles et alimentées en gaz de chauffage de façon à déterminer au moins deux parties dans la première zone dite zone de chauffage, la première partie permettant de porter la charge jusqu'à une température au plus égale à environ 1 500 °C et la deuxième partie, subséquente de la première partie, permettant de maintenir la charge à une température sensiblement égale à la température maximum à laquelle elle a été portée dans ladite première partie. Dans la deuxième zone subséquente à ladite deuxième partie, on refroidit les effluents de la zone de chauffage, puis on recueille les produits formés à l'extrémité de la zone réactionnelle. Il est ainsi possible de déterminer dans la deuxième zone du réacteur un ou plusieurs moyens d'échange de chaleur utilisés comme éléments permettant de refroidir les effluents sortant de la deuxième partie de la zone de chauffage par échange indirect de chaleur et soit de recueillir alors les produits formés à l'extrémité de cette zone de refroidissement ou trempe, soit de prévoir à la suite de cette zone de trempe indirecte une zone de trempe directe puis de recueillir les produits à la sortie de cette zone de trempe directe.

C'est ainsi que dans un mode de réalisation du dispositif de l'invention, le réacteur est constitué (côté première extrémité) d'une première zone dans laquelle certains au moins et de préférence la majorité des moyens d'échange de chaleur de cette première zone forment des moyens de chauffages du ou des réactif(s) introduit(s) dans ledit réacteur, et d'une deuxième zone dans laquelle certains au moins et de préférence la majorité des moyens d'échange de chaleur de cette deuxième zone forment des moyens de refroidissement ou de trempe indirecte des produits issus de ladite première zone.

Selon ces réalisations, les moyens d'échange de chaleur de la première zone alimentés en gaz peuvent être alimentés par des gaz de combustion, à température élevée, issus de préférence d'un brûleur alimenté en combustible et en comburant tel que de l'air, et les moyens d'échange de chaleur de la deuxième zone alimentés en gaz sont chacun alimentés par au moins un gaz à température plus faible que celle des gaz de combustion alimentant ladite première zone, et de préférence par les gaz issus d'un brûleur uniquement alimenté en comburant tel que de l'air.

Dans le cas du vapocraquage d'hydrocarbure qui s'effectue à une température de l'ordre de 800 à 1 300 °C, comme dans celui du couplage thermique déshydrogénant du méthane qui s'effectue à une température de l'ordre de 1 100 à 1 500 °C qui sont deux réactions fortement endothermiques, il est indispensable d'obtenir une densité de flux thermique très importante. Il est nécessaire que l'apport maximum de chaleur soit effectué dans la zone où s'effectuent les réactions endothermiques de craquage et de déshydrogénation ; par ailleurs, compte tenu de la réactivité des produits formés, comme l'acétylène, l'éthylène et/ou le propylène, il est nécessaire d'avoir un temps de contact contrôlé, relativement court, suivi d'une trempe rapide, de façon à obtenir un profil de température de type "carré" et à éviter une trop grande formation de coke.

Les échanges thermiques sont l'un des éléments clé pour ce type de réaction très endothermique où il est nécessaire de transférer des quantités d'énergie très importantes depuis les moyens d'échange de chaleur employés comme moyens de chauffage vers le mélange gazeux contenant au moins un hydrocarbure dénommé ci-après gaz process. Au cours de l'étude préliminaire effectuée par la demanderesse sur les échanges thermiques dans un four à pyrolyse construit selon le modèle utilisé dans la présente invention, on s'est aperçu qu'il n'y a en général que peu d'échange radiatif entre l'enveloppe externe du moyen d'échange de chaleur et le gaz process. En effet, celui-ci est habituellement essentiellement constitué d'un mélange hydrocarbures-gaz de dilution, mélange qui absorbe peu le rayonnement émis par l'enveloppe. Le transfert thermique, entre le gaz process et l'enveloppe, est donc dans le cas envisagé dans la présente invention principalement un transfert par convection. Dans un tel cas, la qualité des échanges thermiques sera directement liée à la surface d'échange disponible, au rapport surface/volume ainsi qu'à la bonne homogénéité en température des moyens d'échange de chaleur.

Ainsi, si la surface d'échange est relativement faible, il sera nécessaire, pour obtenir une température de gaz process donnée correspondant à un taux de conversion préalablement choisi, d'augmenter la température de l'enveloppe du moyen d'échange de chaleur dans des proportions d'autant plus importantes que cette surface est faible, ce qui implique un risque accru de formation de coke.

Les parois participent de manière importante à l'échange thermique, puisqu'elles sont capables d'absorber le rayonnement émis par les enveloppes des moyens d'échange de chaleur et par conséquent les températures de ces enveloppes et des parois ont tendance à s'équilibrer. Il est alors possible d'augmenter notablement la surface d'échange et pratiquement de la doubler en concevant le dispositif de manière particulière. En effet bien que les moyens d'échange de chaleur puissent être disposés en quinconce, il est préférable en vue d'augmenter la surface d'échange, de disposer ces moyens d'échange de chaleur de manière à ce qu'ils soient alignés, ce qui permet de constituer n rangées de m moyens d'échange de chaleur dans le sens de la longueur (pour un nombre total de moyens d'échange de chaleur égal à n x m), on formera ainsi au moins une zone longitudinale et le plus souvent au moins deux zones longitudinales comprenant chacune au moins une et souvent plusieurs nappes de moyens d'échange de chaleur, chaque zone longitudinale étant séparée de la suivante par une paroi en matériau réfractaire. Il est ainsi possible d'augmenter la surface d'échange de chaleur par une surface optimisée telle que par exemple par l'adjonction d'ailettes sur les enveloppes externes des moyens d'échange de chaleur participant au transfert thermique.

Par radiation, la température de ces parois augmente et a tendance à atteindre une valeur très proche de celle des enveloppes externes des moyens d'échange de chaleur. Ces parois participeront donc également au chauffage par convection du gaz process. Ainsi, dans cette forme de réalisation, la surface d'échange étant notablement augmentée, on pourra obtenir la même température de gaz process avec une température des enveloppes externes des moyens d'échange de chaleur et des parois relativement plus faible, ce qui permet en conséquence une diminution de la formation de coke.

Dans une forme particulière de réalisation de l'invention, chaque zone longitudinale comprendra une seule rangée de moyens d'échange de chaleur.

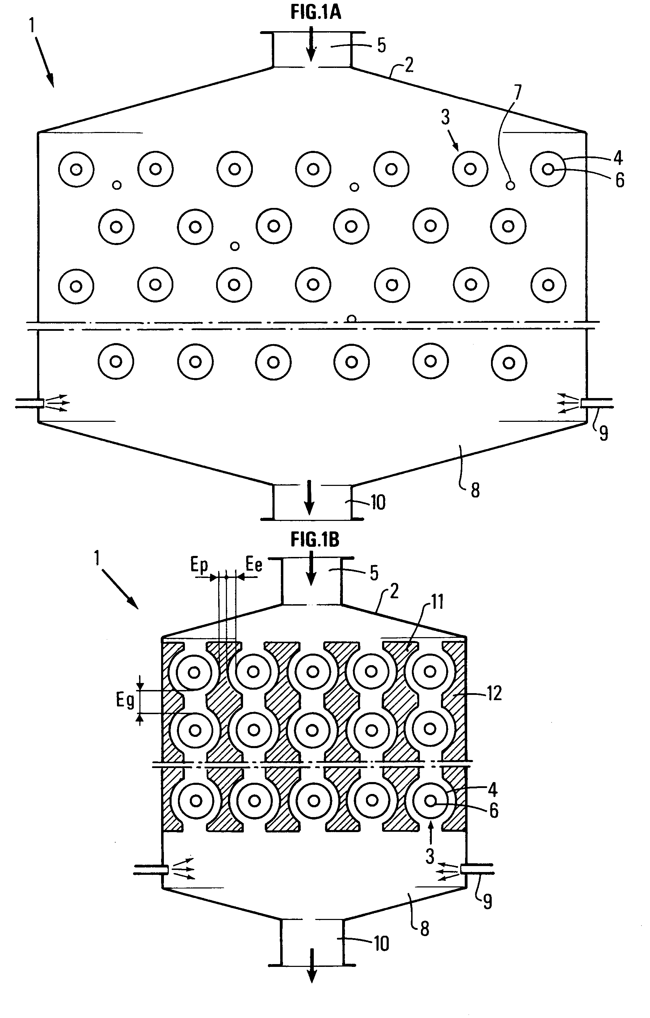

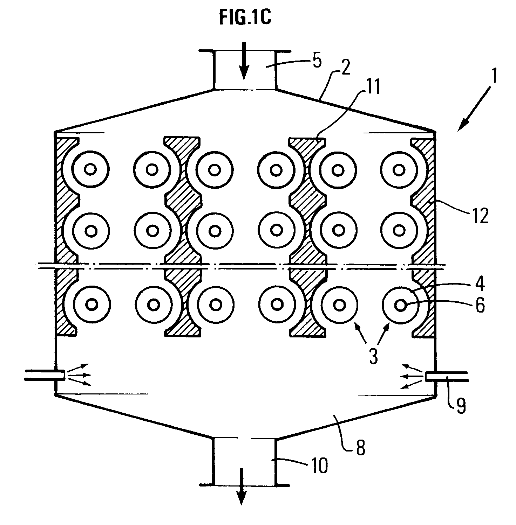

Selon ces deux formes de réalisation, les échanges convectifs entre le gaz process et les parois sont largement augmentés et ils peuvent être encore améliorés en imposant au gaz process des vitesses importantes et en créant des zones de turbulence. L'augmentation de la vitesse du gaz process peut par exemple être obtenue en utilisant des parois dont la forme favorise cette augmentation de vitesse et l'apparition de zones de turbulence. Des parois de forme particulières sont représentées à titre non limitatif sur les figures 1B et 1C.

Les parois sont habituellement en matériau réfractaire. Tout matériau réfractaire peut être utilisé pour réaliser les parois et on peut citer à titre d'exemples non limitatifs la zircone, le carbure de silicium, la mullite et divers bétons réfractaires.

Étant donné qu'il n'est nullement nécessaire d'avoir une étanchéité au niveau des parois, puisque la composition du gaz est pratiquement identique de chaque côté des parois, cette réalisation n'induit qu'une augmentation minime du coût du dispositif. En effet, d'une part il n'est pas nécessaire d'avoir des parois spécialement épaisses, ni une réalisation particulièrement complexe, d'autre part les dimensions globales du réacteur augmentent peu car l'essentiel de la largeur de ce réacteur est due à la largeur des enveloppes externes des moyens d'échange de chaleur. A titre d'exemple, ces enveloppes peuvent avoir une largeur de l'ordre de 165 mm, pour une épaisseur de paroi ayant une valeur de l'ordre de 50 mm, ce qui ne provoque qu'une augmentation de largeur globale du réacteur de l'ordre de 30 %.

Un avantage supplémentaire de cette réalisation comportant des parois est de permettre une réalisation plus simple du réacteur, les parois verticales permettant, outre l'amélioration du transfert thermique par convection, de supporter la voûte du réacteur.

Par ailleurs il est préférable que chaque paroi comporte au moins un moyen permettant d'équilibrer les pressions dans les zones longitudinales situées de part et d'autre de la paroi. A titre d'exemple de moyen simple mais efficace permettant d'équilibrer les pressions, on peut citer la création de zones comportant une ou plusieurs perforations ou de zones poreuses.

La zone réactionnelle peut aussi, selon une autre réalisation, comprendre une série d'éléments dits éléments passifs de transfert de chaleur dont les principales fonctions sont d'augmenter la surface d'échange, de limiter le temps de séjour et d'ajuster la vitesse de passage des réactifs. Ces éléments passifs de transfert de chaleur peuvent être également utilisés en combinaison avec les parois en matériau réfractaire notamment lorsqu'entre deux parois on a plusieurs nappes de moyens d'échange de chaleur. Ces parois tout comme ces éléments passifs de transfert de chaleur, qui dans le cas de la réalisation de réactions endothermiques sont, au moins dans une première partie de la zone réactionnelle, des éléments chauffants participant à l'échange thermique en absorbant, puis en restituant, une partie du rayonnement émis par les moyens d'échange de chaleur.

Selon un autre mode de réalisation, la zone de chauffage et/ou la zone réactionnelle contiennent des éléments de garnissage dont le matériau constitutif est choisi de préférence dans le groupe formé par les matériaux réfractaires. Ces éléments de garnissage peuvent être en matériau du type béton réfractaire, céramiques monolithiques ou fibres céramiques. Ce sont donc soit des éléments sous forme divisée, soit des éléments massifs qui peuvent alors être considérés pour ceux qui se trouvent dans la zone réactionnelle comme les éléments passifs définis ci-devant. On peut introduire simultanément à la fois des éléments sous forme divisée et des éléments massifs. Ces éléments de garnissage sont souvent utilisés pour limiter le temps de séjour et ajuster la vitesse de passage du gaz process (réactifs et/ou produits formés) dans la zone réactionnelle. Ces éléments lorsque ce sont des éléments massifs peuvent également servir de support des parois externes au moins en partie en matériau réfractaire. Ils peuvent aussi avoir des dimensions telles qu'ils ne sont reliés qu'à une paroi externe et servir alors de chicane augmentant la surface d'échange entre le gaz process et les moyens d'échange de chaleur.

Dans une forme particulière de réalisation dont l'un des avantages sera une flexibilité encore accrue du dispositif de l'invention, le réacteur comprendra des moyens d'échange de chaleur formés d'un tube, comprenant une enveloppe non alimentée en gaz ou mélange de gaz, lesdits moyens formant ainsi des éléments passifs de transfert de chaleur.

Dans une forme particulière de réalisation, ce tube peut être un tube en doigt de gant, comprenant une enveloppe extérieure fermée à l'une de ces extrémités et une enveloppe intérieure, sensiblement coaxiale à ladite enveloppe extérieure, ouverte à ces deux extrémités non alimentée en gaz ou mélange de gaz, lesdits moyens formant ainsi des éléments passifs de transfert de chaleur.

Selon l'une des caractéristiques de l'invention, les moyens d'échange de chaleur qui fournissent de la chaleur à la zone de chauffage sont alimentés de façon indépendante, soit isolément, soit par sections transverses, de façon à définir des sections de chauffage le long de la zone de chauffage et à pouvoir ainsi moduler la quantité d'énergie fournie tout au long de cette zone.

La zone de chauffage est habituellement composée de 2 à 20 sections de chauffage, de préférence de 5 à 12 sections. Dans la première partie de cette zone, le mélange gazeux renfermant au moins un hydrocarbure, préalablement chauffé à environ 400 °C à 1 000 °C, est habituellement porté à une température au plus égale à environ 1 500 °C, et avantageusement entre 800 et 1 300 °C (le début de la zone de chauffage est situé à l'endroit où le ou les réactifs sont introduits).

La modulation de ces sections de chauffage est réalisée de façon classique ; les moyens d'échange de chaleur correspondant aux sections précitées sont en général alimentés dans des conditions de débit de gaz combustible et comburant permettant d'obtenir la température souhaitée à l'extérieur de l'enveloppe externe de chaque moyen d'échange de chaleur.

Afin de permettre la régulation de l'ensemble, chaque section de chauffage peut être munie de capteurs de température ; ces capteurs sont disposés dans les espaces où circule la charge, les informations sont transmises au régulateur qui commande les débits de gaz combustible et comburant.

La longueur de la première partie de la zone de chauffage représente habituellement au moins 5 % de la longueur totale de la zone de chauffage, avantageusement au moins 20 % et par exemple de 20 à 90 %.

L'énergie fournie à cette première partie de la zone de chauffage est telle qu'elle génère un fort gradient de température qui permet d'avoir une température moyenne de la charge, sur l'ensemble de la zone de chauffage, relativement élevée. Ce gradient de température est habituellement d'environ 0,5 à environ 25 °C/cm. Dans le cas de la pyrolyse d'hydrocarbures tel que par exemple la pyrolyse de l'éthane, ceci est favorable à la sélectivité en oléfines légères.

Dans la deuxième partie de la zone de chauffage, on module l'énergie fournie aux différentes sections de chauffage de cette zone de façon à ce que la variation de température tout au long de cette zone soit faible, habituellement inférieure à environ 50 °C (+ ou - 25 °C autour de la valeur de la consigne) et avantageusement inférieure à environ 20 °C (+ ou - 10 °C autour de la valeur de la consigne).

Par ailleurs, l'utilisation de différentes sections transversales de chauffage, indépendantes les unes des autres, permet d'apporter, au niveau de la deuxième partie de la zone de chauffage, le maximum d'énergie thermique à l'endroit où s'effectue la plus grande partie des réactions endothermiques, et de maintenir dans le reste de la zone de chauffage une température quasi uniforme.

La longueur de la zone de chauffage est habituellement d'environ 50 à environ 90 % de la longueur totale de la zone réactionnelle.

On obtient, notamment dans les conditions de chauffage ci-dessus, un flux thermique très important à un niveau de température élevé. Ceci implique habituellement un choix particulier pour le matériau constitutif des enveloppes externes des tubes en doigt de gant. A titre d'exemple de matériau utilisable pour la réalisation de ces enveloppes on peut citer les matériaux céramiques, le carbure de silicium, le nitrure de bore, le nitrure de silicium, et la zircone.

Dans une forme de réalisation du dispositif selon l'invention particulièrement avantageuse, certains au moins des moyens d'échange de chaleur (3) comportent un tube alimenté en gaz ou mélange de gaz à l'aide d'un brûleur comportant une enveloppe formant une chambre annulaire autour dudit brûleur dans laquelle le gaz ou le mélange de gaz d'échange de chaleur circule puis est évacué vers l'extérieur de la dite chambre.

Dans une forme préférée de réalisation, certains au moins des moyens d'échange de chaleur (3) comportent un tube en doigt de gant alimenté en gaz ou mélange de gaz à l'aide d'un brûleur comportant une enveloppe formant une chambre annulaire autour dudit brûleur dans laquelle le gaz ou le mélange de gaz d'échange de chaleur provenant de l'espace annulaire entre les deux enveloppes du tube en doigt de gant circule puis est évacué vers l'extérieur de la dite chambre. Dans ce cas l'utilisation d'un brûleur jet à haute vitesse de sortie de gaz entraîne une recirculation efficace des produits gazeux et, par suite une très bonne homogénéité de température sur toute la hauteur du tube enveloppe extérieur dudit tube en doigt de gant.

Le plus souvent, l'enveloppe extérieure du tube en doigt de gant et l'enveloppe formant la chambre annulaire autour du brûleur sont coaxiales et ont le même diamètre intérieur. Habituellement, ces deux enveloppes c'est-à-dire l'enveloppe extérieure du tube en doigt de gant et l'enveloppe formant la chambre annulaire autour du brûleur, sont formées par un seul et unique tube.

L'enveloppe intérieure du tube en doigt de gant comprend généralement sur sa surface cylindrique radialement externe des nervures de centrage dans l'enveloppe extérieure du tube en doigt de gant.

On peut avantageusement employer un moyen d'échange de chaleur dans lequel l'enveloppe intérieure du tube en doigt de gant comprend une pluralité de tronçons de tube disposés bout-à-bout sensiblement alignés axialement et assemblés par des moyens d'assemblage comprenant un organe annulaire formant un manchon entourant coaxialement les tronçons de tube à relier au niveau de la zone de jonction et dont la surface radialement interne comprend au milieu de sa longueur une collerette annulaire radialement saillant vers l'intérieur et s'engageant entre les deux tronçons de tube à relier, les parties de la surface interne situées de par et d'autre de la collerette vont en s'élargissant progressivement vers les extrémités du manchon. Dans ce dernier cas le manchon porte, le plus souvent, sur sa surface cylindrique radialement externe des nervures de centrage de l'enveloppe intérieure du tube en doigt de gant dans l'enveloppe extérieure du tube en doigt de gant.

Selon la présente invention on utilisera avantageusement un tube en doigt de gant tel que celui décrit dans le document de brevet au nom de Gaz de France FR-A-2 616 518 (US 4 850 334) et on pourra aussi utiliser le brûleur jet autorécupérateur décrit dans le document de brevet au nom de Gaz de France FR-B-2 616 520 (US 5 894 006), incorporés comme références.

L'invention sera mieux comprise par la description de quelques modes de réalisation, donnés à titre purement illustratif mais nullement limitatif qui en sera faite ci-après à l'aide des figures annexées, sur lesquelles les organes similaires sont désignés par les même chiffres et lettres de référence. Ces figures concernent une réalisation préférée du réacteur selon l'invention dans le cas de l'utilisation d'au moins un tube en doigt de gant et d'au moins un brûleur jet dans un procédé où sont mises en jeu des réactions endothermiques ou exothermiques.
- Les figures 1A, 1B et 1C représentent une coupe longitudinale d'un réacteur suivant un plan perpendiculaire à l'axe des enveloppes. Dans le cas des figures 1B et 1C ce réacteur comporte des parois séparant une ou plusieurs nappes de moyens d'échange de chaleur.

La figure 2 illustre en coupe longitudinale suivant l'axe d'un moyen d'échange de chaleur, le détail de cet élément lorsque celui-ci est un moyen de chauffage comportant un brûleur jet autorécupérateur.

Sur la figure 1A, on a représenté, selon un mode de réalisation, un réacteur (1) vertical de forme allongée et de section rectangulaire, comprenant un distributeur (2) permettant d'alimenter par un orifice d'entrée (5) le réacteur en mélange gazeux réactionnel. Ce dernier, qui contient par exemple en volume 50 % de méthane et 50 % de gaz de dilution a été préchauffé dans une zone de préchauffage conventionnelle, non représentée sur la figure, de préférence par convection. Le réacteur comprend une pluralité de moyens d'échange de chaleur (3) comprenant chacun une enveloppe externe (4) et un tube interne sensiblement coaxial (6), disposés en nappes parallèles et formant dans un plan (plan de la figure) un faisceau à pas triangulaire. Ces nappes définissent des sections de chauffage ou de refroidissement transversales sensiblement perpendiculaires à l'axe du réacteur défini selon la direction d'écoulement de la charge.

Ces sections de chauffage sont alimentées en calories par la circulation d'un gaz ou d'un mélange de gaz introduit dans le tube interne (6) et évacué dans l'espace annulaire entre le tube interne (6) et l'enveloppe externe (4), de façon indépendante. Des sondes pyrométriques à thermocouple (7) sont logées dans les espaces où circule la charge entre les moyens d'échange de chaleur (3) à l'extérieur de l'enveloppe (4) desdits moyens et permettent la régulation automatique de la température de chaque section de chauffage, par un dispositif classique de régulation non représenté sur la figure.

Dans la première partie de la zone de chauffage et dans le cas de la conversion thermique du méthane, les moyens d'échange de chaleur sont chauffés de façon à ce que la température de la charge passe rapidement de la température de préchauffage (par exemple 750 ou 1 000 °C) à 1 200 °C environ ; cette zone de chauffage progressif représente en général environ 25 % de la longueur totale de la zone de chauffage le mélange gazeux circule ensuite dans la deuxième partie de la zone de chauffage où l'on maintient généralement la température à une valeur constante sensiblement égale à celle atteinte à la fin de la première zone de chauffage, soit en général 1 200 °C environ. A cet effet, on module la puissance de chauffe fournie à plusieurs sections de chauffage qui constituent la deuxième partie de la zone de chauffage ; on parvient ainsi à obtenir une variation de température ne dépassant pas environ 10 °C autour de la valeur de la consigne. La longueur de cette deuxième partie de zone de chauffage représente environ 75 % de la longueur totale de la zone de chauffage.

A la sortie de la zone de chauffage, les effluents de la réaction sont refroidis dans une zone de refroidissement (8). Ils sont mis en contact avec un agent de trempe tel que par exemple du propane introduit par l'intermédiaire d'injecteurs (9), de trempe, disposés à la périphérie du réacteur (1) et reliés à une source extérieure de propane non représentée. L'ensemble des gaz effluents est refroidi à une température d'environ 500°C et recueilli par un orifice de sortie (10) à l'extrémité du réacteur(1).

Selon un autre mode, les effluents peuvent être au moins en partie refroidis en circulant à travers une zone, située après la deuxième partie de la zone de chauffage, dans laquelle les éléments d'échange de chaleur sont des éléments de refroidissement comportant l'alimentation du tube interne (6) par un gaz ou un mélange de gaz relativement froid introduit dans ledit tube et évacué dans l'espace annulaire entre le tube interne (6) et l'enveloppe externe (4). Il est possible mais habituellement non indispensable de poursuivre ce refroidissement par trempe directe dans la zone (8) comme décrit ci-devant.

Sur la figure 1B, on a représenté, selon un mode de réalisation, un réacteur (1) vertical de forme allongée et de section rectangulaire, qui diffère du réacteur représenté sur la figure 1A par le fait qu'il comporte des moyens d'échange de chaleur disposés en rangées de nappes sensiblement parallèles et formant dans un plan (plan de la figure) un faisceau à pas carré. Ces rangées sont séparées les unes des autres par des parois (11) avantageusement en matière céramique sensiblement parallèles à l'axe du réacteur. Ces parois ont une forme, adaptée à créer des turbulences, comportant des alvéoles au niveau de chaque moyen d'échange de chaleur (3). Ce réacteur comporte sur la face interne de ses parois externes une partie saillante (12), avantageusement en matière céramique, ayant une forme, adaptée à créer des turbulences, comportant des alvéoles au niveau de chaque moyen d'échange de chaleur (3). La distance Eg séparant deux moyens d'échange de chaleur (3) voisins n'est pas très critique mais reste cependant assez faible pour des questions de temps de séjour et de densité de flux thermique à assurer. Elle est habituellement d'environ 2 mm à environ 100 mm. Les rangées de moyens d'échange de chaleur (3) sont séparées par une paroi par exemple en béton réfractaire à base d'alumine électrofondue. La distance Ee entre les moyens d'échange de chaleur (3) et les parois ou dimension des passages est habituellement assez faible de manière à assurer un bon contact des gaz avec la surface externe de l'enveloppe (4) des moyens d'échange de chaleur (3). Elle est habituellement d'environ 1 mm à environ 100 mm et de préférence d'environ 2 mm à environ 50 mm. Les parois ont dans leur partie la plus mince une épaisseur Ep relativement faible mais suffisante pour assurer une bonne tenue mécanique de ces parois. Elle est habituellement d'environ 2 mm à environ 300 mm et de préférence d'environ 5 mm à environ 50 mm.

Le mode de réalisation schématisé sur la figure 1C ne diffère de celui schématisé sur la figure 1B qu'en ce que plusieurs nappes de moyens d'échange de chaleur (3) sont situées entre deux parois (11).

La figure 2 illustre en coupe longitudinale suivant l'axe d'un moyen d'échange de chaleur, le détail de cet élément lorsque celui-ci est un moyen de chauffage comportant un brûleur jet autorécupérateur.

Ce brûleur comprend essentiellement un tube foyer 13 en céramique thermomécanique qui délimite la chambre de combustion 14 du brûleur et présente une forme simple avec un rétreint 19 à l'extrémité de sortie des gaz brûlés, pour donner une forte impulsion aux produits de combustion à leur sortie du brûleur. Du côté opposé à la sortie 17 définie par le rétreint 19, la chambre de combustion 14 est fermée par un disque 15 de préférence transparent. Un élément tubulaire 16 entoure coaxialement le tube foyer 13. Cet élément tubulaire 16 comprend à son extrémité située du côté de la sortie 17 des gaz brûlés un rétrécissement radial qui forme une butée 18 de positionnement axial du tube foyer 13 qui entoure coaxialement le rétreint 19 de celui-ci. Le tube foyer 13 prend appui sur cette butée par un épaulement radial annulaire, pratiqué dans le rétreint 19 avec interposition d'un joint d'étanchéité. L'élément tubulaire 16 entoure également coaxialement un tube 20 qui est placé en prolongation axiale du tube foyer 13, du côté du disque de fermeture 15 de la chambre de combustion. Le tube 20 présente sensiblement le même diamètre que le tube foyer 13.

Sur la figure 2, qui montre un brûleur jet conformé en brûleur à récupération, l'élément tubulaire 16 est réalisé sous forme d'un échangeur de chaleur en alliage réfractaire à ailettes 21. Cet échangeur de chaleur est constitué à partir de tronçons d'une certaine longueur, par exemple de 50 mm, qui sont assemblés entre eux. Un tube enveloppe extérieur 4 et un tronçon de tube 43 qui est disposé dans le prolongement axial du tube 4 entourent coaxialement l'échangeur de chaleur 16. A leur extrémité en regard, les tubes 4 et 43 sont pourvus de brides respectivement 24 et 25 qui permettent le raccordement des tubes entre eux, avec interposition d'un joint non indiqué et la fixation du brûleur à la paroi du réacteur 1 schématiquement représentée en 44. L'espace délimité par les tubes 4 et 43 est fermé, du côté opposé au tube foyer 13 par un élément tubulaire d'enveloppe 29 qui est situé dans le prolongement axial du tube 43 et est fermé à son extrémité libre, par une paroi 30 perpendiculaire à l'axe de l'ensemble tubulaire. Les extrémités en regard du tube 43 et de l'élément d'enveloppe 29 sont pourvues de brides de raccordement 31, 32. L'échangeur de chaleur 16 est maintenu à son extrémité opposée à la butée 18 pour le positionnement du tube foyer entre les brides 31 et 32, par des éléments 33 faisant radialement saillies vers l'extérieur. Le tronçon du tube 43 est muni d'un piquage 35 d'évacuation des gaz brûlés à l'extérieur du réacteur, tandis que l'élément d'enveloppe 29 est équipé d'un piquage 36 pour l'arrivée de l'air de combustion. On constate que l'échangeur de chaleur 16 sépare les flux d'air de combustion entrant dans le brûleur en 36 et les gaz brûlés qui circulent à contre-courant et sortent du brûleur par le piquage 35.

L'arrivée du ou des gaz combustibles est assurée par un tube d'amenée 37 qui s'étend dans l'axe du tube 20 depuis la paroi 30 de l'élément enveloppe 29 dans la chambre de combustion 14 en traversant le disque 15. Le ou les gaz combustibles pénètrent dans la chambre 14 par des orifices 38 par exemple au nombre de six, qui sont répartis angulairement sur le conduit formant canne de gaz 37, près de son extrémité. La canne 37 porte à son extrémité un dispositif d'allumage à haute tension. Une électrode de masse représentée en 39 est soudée sur la canne tandis qu'une électrode haute tension désignée en 40 est située dans l'axe de celle-ci. Elle est par exemple constituée d'une tige en alliage réfractaire placée dans une gaine en alumine. L'air ou le gaz comburant pénètre dans la chambre de combustion 14 par des trous P pratiqués dans le tube foyer 13 qui sont habituellement situés dans un même plan sensiblement perpendiculaire à l'axe du foyer. Le plus souvent, ce tube foyer comprend plusieurs plans d'arrivée d'air ou de gaz comburant et les trous d'entrée d'air ou de gaz comburant sont pratiqués de manière à ce que l'air ou le gaz comburant pénètre dans la chambre de combustion 14 selon des trajectoires sensiblement tangentielles.

Les gaz brûlés sortent de la chambre de combustion 14 par la sortie 17 et pénètrent directement dans le tube interne comprenant, selon la réalisation schématisée sur cette figure, deux tronçons 6a et 6b et formant l'enveloppe intérieure du tube en doigt de gant formant un moyen d'échange de chaleur 3. Ce moyen d'échange de chaleur 3 est dans cette réalisation un tube radiant en doigt de gant à recirculation, qui comprend un tube enveloppe externe 4 qui forme également l'enveloppe extérieure du dispositif brûleur décrit ci-devant.

Les tubes ou enveloppes du moyen d'échange de chaleur 3 sont le plus souvent en matière céramique thermomécanique. On ne sortirait cependant pas du cadre de la présente invention en utilisant pour leur réalisation une autre matière ayant des caractéristiques de résistance mécanique et chimiques compatibles avec l'utilisation envisagée. En particulier l'enveloppe extérieure 4 pourrait être en alliage réfractaire. Le tube interne du moyen d'échange de chaleur 3 peut être en un seul tronçon, mais se compose le plus souvent de plusieurs tronçons, et par exemple de 2 à 12 tronçons, axialement alignés et reliés entre eux par des moyens d'articulation, c'est-à-dire de moyens de liaison souple. On constate que les tronçons de tube 6a et 6b sont disposés bout-à-bout. Chacun des moyens de liaison est réalisé sous forme d'un manchon 41, par exemple en céramique, entourant coaxialement les extrémités en bout-à-bout des deux tronçons de tube 6a et 6b reliés. La surface radialement interne du manchon 41 porte, avantageusement au milieu de sa longueur, une collerette annulaire 42 qui fait radialement saillie vers l'intérieur et s'engage entre les deux extrémités des tronçons à assembler. Le diamètre interne de la collerette correspond essentiellement au diamètre interne des tronçons de tube 6a et 6b, tandis que le diamètre externe de la collerette est un peu plus grand que le diamètre externe des tronçons de façon à laisser subsister un certain jeu radial tenant compte d'un désaxage des tronçons tout en assurant l'étanchéité de la jonction.

Dans une forme de réalisation non schématisée sur la figure 2, les parties de surface interne du manchon 41 situées de part et d'autre de la collerette 42 présentent un diamètre qui augmente progressivement en direction de l'extrémité des manchons. L'augmentation du diamètre est déterminée de façon à permettre au tronçon 6a ou 6b de suivre, par un désaxage axial approprié pouvant atteindre quelques degrés, le mouvement de fluage du tube enveloppe 4 en particulier lorsque celui-ci est en alliage réfractaire. Ce dernier peut être ainsi réalisé en un alliage réfractaire, c'est-à-dire en un matériau ayant un coefficient de dilatation et un comportement à haute température très différent du matériau constituant le tube interne 6 (figure 1a, 1b et 1c) (6a et 6b figure 2).

Chaque manchon 41 comporte sur sa surface cylindrique externe des nervures de centrage (non schématisées sur les figures) dans l'enveloppe externe 4. Les premier et dernier tronçons du tube 6 comprennent au niveau de leur extrémité libre des nervures de centrage 45 et peuvent comprendre des découpes ou ouïes en forme de U pratiquées dans la paroi cylindrique des tronçons entre deux nervures de centrage.

Le tube interne 6 est ouvert à ses deux extrémités, ce qui permet la circulation des gaz brûlés sortant de la chambre de combustion 14 dans ce tube et dans l'espace annulaire créé entre le tube interne 6 et le tube enveloppe 4 de la manière indiquée par des lignes fléchées, le tube enveloppe étant fermé à son extrémité éloignée du brûleur. La présence des ouïes favorise la circulation des gaz de combustion. Ainsi le tube radiant peut être utilisé en position verticale même si le dernier tronçon était en appui sur le fond de l'enveloppe 4, après un déplacement axial de ce tronçon ou de plusieurs tronçons. A cette fin la demi-longueur axiale d'un manchon est supérieure à la somme des jeux axiaux entre le tube et les éléments adjacents et les différents tronçons. Étant donné que les tronçons de tube 6 présentent un diamètre interne et externe constant sur toute leur longueur, on peut adapter le tube 6 à toute longueur utile par simple découpe de tronçons ou d'un tronçon à la longueur désirée ou construire le tube à partir d'un certain nombre de modules standards de différente longueur.

Il est souvent préférable que le brûleur soit totalement inclus dans l'une des parois du réacteur, comme cela est schématisé sur la figure 2, mais on ne sortirait pas du cadre de la présente invention si tel n'était pas le cas. De même la position de brûleur est en général choisie de manière à ce que le nez du brûleur, c'est-à-dire la sortie 17 des gaz de combustion se situe à proximité immédiate de la paroi interne 44 du réacteur et de préférence au niveau de cette paroi ou légèrement à l'intérieur de la zone entre les deux parois du réacteur.

Les exemples qui suivent servent à illustrer l'invention mais ne peuvent en aucun cas limiter sa portée. Ils montrent de façon particulièrement claire la flexibilité du dispositif objet de la présente invention.

### EXEMPLE 1

On utilise un réacteur horizontal à trempe directe, de longueur totale utile de 7 m et de section rectangulaire de 1,5 m par 1,6 m dont la configuration est similaire à celle schématiquement représentée sur la figure 1B.

Les moyens d'échange de chaleur (3) comprennent, pour ce qui est des moyens de chauffage de ce réacteur, un brûleur dont les gaz de combustion alimentent l'enveloppe intérieure (6) d'un tube en doigt de gant formant chacun un élément chauffant radiant (3) en carbure de silicium.

Ces éléments chauffants sont disposés perpendiculairement au sens de circulation de la charge (verticalement), en nappes parallèles. Les éléments chauffants (3) en céramique ont une longueur de 1,5 m et un diamètre extérieur de 165 mm ; la distance (Eg) séparant deux éléments chauffants voisins est de 36 mm. Le réacteur comprend cinq rangées longitudinales, séparées par une paroi (11) en béton réfractaire à base d'alumine électrofondue. La distance (Ee) entre les éléments chauffants (3) et la paroi (11 ou 12) ou dimension des passages est de 18 mm. Chaque rangée comprend 30 éléments chauffants (3). Les parois (11) et (12) ont dans leur partie la plus mince une épaisseur (Ep) de 99 mm.

La première partie de chaque rangée, longue de 1.4 m, comprend 7 éléments chauffants (3) par rangée ; dans cette partie, la charge introduite par l'orifice d'entrée (5), préchauffée à 1 000 °C, est portée à 1 200 °C. Cette zone comporte une régulation thermique par l'intermédiaire de thermocouples (7 figure 1A) disposés dans les espaces où circule la charge.

La deuxième partie de chaque rangée, adjacente à ladite première partie, est longue de 4,6 m ; elle est constituée de 23 éléments chauffants (3), disposés de la même façon que dans la première partie de ladite rangée. Chaque élément chauffant (3) comporte un moyen de régulation thermique, permettant d'assurer le maintien de la température dans toute cette zone à 1 200 °C plus ou moins 10 °C.

Les gaz effluents sortant de ladite deuxième partie sont refroidis à 800 °C par trempe directe dans la zone (8) de 1 mètre de long faisant suite à ladite deuxième partie. La trempe est effectuée par introduction d'une coupe C4 de vapocraquage par l'intermédiaire d'injecteurs (9).

La charge est constituée de méthane contenant 2% en poids d'éthane dilué avec de l'hydrogène dans un rapport volumique (hydrocarbures totaux sur hydrogène) de 1,1 : 1. Ce mélange est préchauffé à 1 000 °C et craqué à 1 200 °C dans le réacteur ci-avant. La pression absolue du mélange de gaz à la sortie du réacteur est de 0,140 MPa.

Après refroidissement à température ambiante, pour 500 kmoles de mélange, on obtient 22 kmoles de mélange éthylène/acétylène contenant 70 % en mole d'acétylène.

### EXEMPLE 2

On utilise le même réacteur et une charge constituée d'un mélange butane/isobutane contenant 45 % en mole d'isobutane.

La première partie de chaque rangée, longue de 2,8 m, comprend 14 éléments chauffants par rangée ; dans cette rangée, la charge introduite par l'orifice d'entrée (5), préchauffée à 400 °C, est portée à 900 °C. Cette zone comporte une régulation thermique par l'intermédiaire de thermocouples (7 figure 1A) disposés dans les espaces où circule la charge.

La deuxième partie de chaque rangée, adjacente à ladite première partie, est longue de 0,8 m ; elle est constituée de 4 éléments chauffants, disposés de la même façon que dans la première partie de la rangée. Chaque élément chauffant (3) comporte un moyen de régulation thermique, pour chauffer la charge de 900 °C à 1 000 °C et assurer le maintien à cette température dans toute cette zone à 1 000 °C plus ou moins 10 °C.

Il reste disponible dans chaque rangée 12 moyens d'échange de chaleur (3). Ces moyens d'échange de chaleur (3) sont utilisés pour faire une trempe indirecte des gaz de 1 000 °C à 840 °C. A cet effet, on coupe l'arrivée de gaz combustible dans le brûleur et on règle le débit d'air de manière à effectuer la trempe. La chaleur résiduelle est récupérée par échange direct par introduction d'une coupe C4 de vapocraquage par l'intermédiaire d'injecteurs (9).

La charge est constituée d'un mélange butane/isobutane dans une proportion molaire 45 moles disobutane pour 100 moles de mélange dilué avec de l'azote dans un rapport volumique (hydrocarbures totaux sur azote) de 1 : 3. Ce mélange est préchauffé à 400 °C et craqué à 1 000 °C dans le réacteur décrit ci-devant. La pression absolue du mélange de gaz mesurée en sortie de réacteur est de 0,140 MPa. Après refroidissement à température ambiante, pour 250 kmoles de mélange butane/isobutane et azote, on obtient 22 kmoles de mélange éthylène/acétylène contenant 37 % en mole d'acétylène.

## Revendications

1. Dispositif comprenant un réacteur (1) de forme allongée selon un axe, de préférence à section carrée ou rectangulaire, comportant à une première extrémité au moins un moyen d'alimentation en au moins un réactif, à l'extrémité opposée au moins un moyen d'évacuation des effluents produits, ledit réacteur comprenant dans une première zone (côté première extrémité) une pluralité de moyens d'échange de chaleur (3), lesdits moyens d'échange de chaleur (3), sensiblement parallèles entre eux, étant disposés en nappes sensiblement parallèles perpendiculaires à l'axe du réacteur de façon à définir entre lesdits moyens et/ou les nappes formées par ces moyens, des espaces ou passages pour la circulation du ou des réactifs et/ou des effluents, lesdits moyens d'échange de chaleur (3) étant adaptés à échanger de la chaleur dans lesdits passages par sections transversales successives, indépendantes et sensiblement perpendiculaires à l'axe du réacteur, ledit dispositif étant caractérisé en ce que certains au moins des moyens d'échange de chaleur (3) comprennent des éléments tubulaires d'échange de chaleur, chacun comportant au moins une enveloppe connectée à un brûleur à gaz pour fournir un gaz de combustion ou un mélange de gaz de combustion en tant que gaz ou mélange de gaz d'échange de chaleur, et au moins un moyen d'évacuation à l'extérieur du réacteur dudit gaz ou mélange de gaz ayant échangé de la chaleur avec le ou les réactifs et/ou effluents, lesdits brûleurs à gaz étant raccordés à des moyens d'alimentation en gaz combustible et en gaz comburant et des moyens d'asservissement des brûleurs et de modulation de la quantité de gaz fourni à ces brûleurs sont interposés entre lesdits moyens d'alimentation et lesdits brûleurs, pour que les moyens d'échange de chaleur soient alimentés soit en produit de combustion issus des brûleurs, soit en gaz comburant exclusivement.

2. Dispositif selon la revendication 1 dans lequel le réacteur comprend dans une deuxième zone (8) (côté extrémité opposée) contiguë à la première zone des moyens de refroidissement (9) des effluents reliés à au moins un moyen d'alimentation en fluide de refroidissement.

3. Dispositif selon la revendication 1 dans lequel le réacteur comporte des moyens d'asservissement et de modulation de l'échange de chaleur reliés audits moyens d'échange de chaleur (3).

4. Dispositif selon l'une des revendications 1 à 3 dans lequel certains au moins desdits moyens d'échange de chaleur (3) sont formés par un tube comprenant une enveloppe de forme allongée le long d'un axe.

5. Dispositif selon l'une des revendications 1 à 4 dans lequel certains au moins desdits moyens d'échange de chaleur (3) comportent un tube alimenté en gaz ou mélange de gaz à l'aide d'un brûleur comportant une enveloppe formant une chambre annulaire autour dudit brûleur dans laquelle le gaz ou le mélange de gaz d'échange de chaleur circule puis est évacué vers l'extérieur de la dite chambre.

6. Dispositif selon l'une des revendications 1 à 5 dans lequel certains au moins desdits moyens d'échange de chaleur (3) comportent un tube en doigt de gant, comprenant une enveloppe extérieure fermée à l'une de ces extrémités et une enveloppe intérieure, sensiblement coaxiale à ladite enveloppe extérieure, ouverte à ces deux extrémités et alimentée en gaz ou mélange de gaz d'échange de chaleur par l'une de ces extrémités, ledit gaz ou mélange de gaz d'échange de chaleur étant évacué par l'autre extrémité dans l'espace libre entre les deux enveloppes.

7. Dispositif selon la revendication 6 dans lequel certains au moins desdits moyens d'échange de chaleur (3) comportent un tube en doigt de gant alimenté en gaz ou mélange de gaz à l'aide d'un brûleur comportant une enveloppe formant une chambre annulaire autour dudit brûleur dans laquelle le gaz ou le mélange de gaz d'échange de chaleur provenant de l'espace annulaire entre les deux enveloppes du tube en doigt de gant circule puis est évacué vers l'extérieur de la dite chambre, l'enveloppe extérieure du tube en doigt de gant et l'enveloppe formant la chambre annulaire autour du brûleur étant de préférence coaxiales et ayant de préférence le même diamètre intérieur.

8. Dispositif selon la revendication 6 ou 7 dans lequel l'enveloppe extérieure du tube en doigt de gant et l'enveloppe formant la chambre annulaire autour du brûleur sont formées par un seul et unique tube.

9. Dispositif selon l'une des revendications 6 à 8 dans lequel l'enveloppe intérieure du tube en doigt de gant comprend sur sa surface radialement externe des nervures de positionnement dans l'enveloppe extérieure du tube en doigt de gant.

10. Dispositif selon l'une des revendications 6 à 9 dans lequel l'enveloppe intérieure du tube en doigt de gant comprend une pluralité de tronçons de tube disposés bout-à-bout sensiblement alignés axialement et assemblés par des moyens d'assemblage comprenant un organe annulaire formant un manchon entourant coaxialement les tronçons de tube à relier au niveau de la zone de jonction et dont la surface radialement interne comprend au milieu de sa longueur une collerette annulaire radialement saillant vers l'intérieur et s'engageant entre les deux tronçons de tube à relier, les parties de la surface interne situées de par et d'autre de la collerette vont en s'élargissant progressivement vers les extrémités du manchon.

11. Dispositif selon la revendication 10 dans lequel le manchon porte sur sa surface cylindrique radialement externe des nervures de centrage de l'enveloppe intérieure du tube en doigt de gant dans l'enveloppe extérieure du tube en doigt de gant.

12. Dispositif selon l'une des revendications 1 à 11 dans lequel le réacteur comprend des moyens supplémentaires d'échange de chaleur formés d'un tube, comprenant une enveloppe non alimentée en gaz ou mélange de gaz, lesdits moyens formant ainsi des éléments passifs de transfert de chaleur.

13. Dispositif selon l'une des revendications 1 à 12 dans lequel le réacteur (côté première extrémité) comprend la première zone dans laquelle au moins une partie et de préférence la majeure partie des moyens d'échange de chaleur de cette première zone forment des moyens de chauffages du ou des réactif(s) introduit(s) dans ledit réacteur, et la deuxième zone dans laquelle au moins une partie et de préférence la majeure partie des moyens d'échange de chaleur de cette deuxième zone forment des moyens de refroidissement ou de trempe indirecte des produits issus de ladite première zone.

14. Dispositif selon la revendication 13 dans lequel les moyens d'échange de chaleur de la première zone alimentés en gaz sont chacun alimentés par des gaz de combustion, à température élevée, issus d'un brûleur alimenté en combustible et en comburant tel que de l'air, et les moyens d'échange de chaleur de la deuxième zone alimentés en gaz sont chacun alimentés par au moins un gaz à température plus faible que celle des gaz de combustion alimentant ladite première zone, et de préférence par les gaz issus d'un brûleur uniquement alimenté en comburant tel que de l'air.

15. Dispositif selon l'une des revendications 6 à 14 dans lequel les tubes en doigt de gant comportent des enveloppes dont le rapport de l'aire interne (Sₑ) de la section droite du tube formant l'enveloppe extérieure du tube en doigt de gant à l'aire externe (sᵢ) de la section droite du tube formant l'enveloppe intérieure du tube en doigt de gant est d'environ 1,4 : 1 à environ 25 : 1.

## Claims

1. An apparatus comprising a reactor (1) which is elongate along one axis, preferably of square or rectangular cross section, comprising at least one supply means for at least one reactant at one extremity, and at least one evacuation means at the other extremity for the effluents produced, said reactor comprising, in a first song (near the first extremity), a plurality of heat exchange means (3) which are substantially parallel to each other, said heat exchange means (3) being disposed in substantially parallel layers perpendicular to the reactor axis to define spaces or passages between said means and/or layers formed by said means for the circulation of reactant(s) and/or effluents, said heat exchange means (3) being adapted to exchange heat in said passages through successive transverse sections, which are independent and substantially perpendicular to the reactor axis, said apparatus being characterised in that at least some of the heat exchange means (3) comprise tubular elements for heat exchange, each comprising one shell connected to a gas burner for supplying a combustion gas or a combustion gas mixture as heat exchange gas or gas mixture, and at least one evacuation means for evacuating said gas or gas mixture which has exchanged heat with the reactant(s) and/or effluents from the reactor, said gas burners being connected to means for supplying fuel gas and oxidiser gas and means for controlling the burners and adjusting the quantity of gas supplied to the burners which are interposed between said supply means and said burners so that the heat exchange means are supplied either with the combustion product from the burners or exclusively with oxidiser gas.

2. An apparatus according to claim 1 wherein the reactor comprises in a second zone (8) (near the opposite extemity) which is contiguous with the first zone, means (9) for cooling the effluent connected to at least one means for supplying cooling fluid.

3. An apparatus according to claim 1, wherein the reactor comprises beans for controlling and adjusting heat exchange connected to said beat exchange means (3).

4. An apparatus according to any one of claims 1 to 3, wherein at least some of said heat exchange means (3) are formed by a tube comprising a shell which is elongate along one axis.

5. An apparatus according to any one of claims 1 to 4, wherein at least some of said heat exchange means (3) comprise a tube supplied with a gas or mixture of gases from a burner, comprising a shell forming an annular chamber around said burner in which the heat exchange gas or gas mixture circulates and is then evacuated from said chamber.

6. An apparatus according to any one of claims 1 to 5, wherein at least some of said heat exchange means (3) comprise a blind tube including an outer shell which is closed at one extremity and an inner shell which is substantially coaxial with said outer shell, open at its two extremities and supplied with said heat exchange gas or gas mixture at one of its extremities, said heat exchange gas or gas mixture being evacuated by the other extremity into the free space between the two shells.

7. An apparatus according to claim 6, wherein at least some of said heat exchange means (3) comprise a blind tube supplied with a gas or gas mixture from a burner, comprising a shell forming an annular chamber around said burner in which the heat exchange gas or gas mixture from the annular space between the two shells of the blind tube can circulate and then be evacuated from said chamber, the outer shell of the blind tube and the shell forming the annular chamber around the burner preferably being coaxial and preferably having the same internal diameter.

8. An apparatus according to claim 6 or claim 7, wherein the outer shell of the blind tube and the shell forming the annular chamber around the burner are formed by a single tube.

9. An apparatus according to any one of claims 6 to 8, wherein the inner shell of the blind tube has positioning ribs on its radially outer surface to position it in the outer shell of the blind tube.

10. An apparatus according to any one of claims 7 to 9, wherein the inner shell of the blind tube includes a plurality of tube sections disposed end-to-end and substantially axially aligned and assembled by assembling means comprising an annular member forming a sleeve which coaxially surrounds the tube sections to connect them at a joint zone and in which the radially inner surface comprises, at the mid point of its length, an annular flange which projects radially inwardly and engages between the two tube sections to be connected, the portions of the inner surface situated either side of the flange widening gradually towards the extremities of the sleeve.

11. An apparatus according to claim 10, wherein the sleeve has centring ribs on the radially outer cylindrical surface for centring the inner shell of the blind tube in the outer shell of the blind tube.

12. An apparatus according to any one of claims 1 to 11, wherein the reactor comprises additional heat exchange means in the form of a tube comprising a shell which is not supplied with gas or a gas mixture, said means thus forming passive heat transfer elements.

13. An apparatus according to any one of claims 1 to 12, wherein the reactor (near the first extremity) comprises a first zone in which at least a portion, preferably a major portion of the heat exchange means of this first zone form heating means for the reactant(s) introduced into said reactor, and a second zone in which at least a portion, preferably a major portion of the heat exchange means of this second zone form cooling or indirect quenching means for the products from said first zone.

14. An apparatus according to claim 13, wherein said heat exchange means in the first zone are each supplied with the combustion gases, at high temperature, from a burner supplied with fuel and an oxidiser such as air, and the heat exchange means in the second zone are each supplied with at least a gas at a lower temperature to that of the combustion gases supplying said first zone, preferably by the gases from a burner which is supplied only with an oxidiser such as air.

15. An apparatus according to any one of claims 6 to 14, wherein the blind tubes comprise shells in which the ratio of the inner area (Sₑ) of the cross section of the tube forming the outer shell of the blind tube to the outer area (sᵢ) of the cross section of the tube forming the inner shell of the blind tube is about 1.4:1 to about 25:1.

## Patentansprüche

1. Vorrichtung, die einen Reaktor (1) mit längs einer Achse länglicher Gestalt, bevorzugt mit quadratischem oder rechteckigem Querschnitt, umfaßt und an einem ersten Ende wenigstens ein Mittel zur Speisung mit wenigstens einem Reaktionsteilnehmer, am gegenüberliegenden Ende wenigstens ein Mittel zum Abzug der erzeugten Abströme aufweist, wobei dieser Reaktor in einer ersten Zone (auf der Seite des ersten Endes) eine Vielzahl von Wärmeaustauschermitteln (3) umfaßt, diese Wärmeaustauschermittel (3), die im wesentlichen parallel untereinander laufen, in im wesentlichen parallelen Bahnen senkrecht zur Achse des Reaktors angeordnet sind, derart, daß zwischen diesen Mitteln und/oder den durch diese Mittel gebildeten Bahnen Räume oder Durchlässe für die Zirkulation des oder der Reaktionsteilnehmer und/oder der Abströme definiert werden, wobei diese Wärmeaustauschermittel (3) so ausgelegt sind, daß sie Wärme in diesen Durchlässen in aufeinanderfolgenden transversalen unabhängigen Abschnitten und im wesentlichen senkrecht zur Achse des Reaktors austauschen, wobei die Vorrichtung sich dadurch auszeichnet, daß wenigstens gewisse der Wärmeaustauschermittel (3) röhrenförmige Wärmeaustauscherelemente umfassen, von denen jedes wenigstens einen mit einem Gasbrenner verbundenen Mantel umfaßt, um ein Verbrennungsgas oder ein Gemisch aus Verbrennungsgas als Wärmeaustauschgas oder Wärmeaustauschgasgemisch zu liefern und wenigstens ein Mittel, um nach außerhalb des Reaktors dieses Gas oder Gasgemisch abzuziehen, das Wärme mit dem oder den Reaktionsteilnehmern und/oder Abströmen ausgetauscht hat, wobei diese Gasbrenner verbunden sind mit Mitteln zur Speisung mit Verbrennungsgas und mit die Verbrennung bewirkendem Gas und wobei Regelmittel für die Brenner und zur Modulation des diesen Brennern gelieferten Gases zwischen diesen Speisemitteln und diesen Brennern zwischengeschaltet sind, damit die Wärmeaustauschermittel entweder mit einem aus den Brennern stammenden Verbrennungsprodukt oder mit einem die Verbrennung bewirkenden Mittel ausschließlich gespeist werden.

2. Vorrichtung nach Anspruch 1, bei dem der Reaktor in einer zweiten Zone (8) (am gegenüberliegenden Ende) angrenzend an die erste Zone Kühlmittel (9) für die Abströme umfaßt, die mit wenigstens einem Mittel zur Speisung mit Kühlfluid verbunden sind.

3. Vorrichtung nach Anspruch 1, bei dem der Reaktor Regel- und Modulationsmittel für den Wärmeaustausch umfaßt, die mit diesen Wärmeaustauschermitteln (3) verbunden sind.

4. Vorrichtung nach einem der Ansprüche 1 bis 3, bei der wenigstens gewisse dieser Wärmeaustauschermittel (3) durch ein Rohr gebildet sind, das einen Mantel mit längs einer Achse länglicher Form umfaßt.

5. Vorrichtung nach einem der Ansprüche 1 bis 4, bei dem wenigstens gewisse dieser Wärmeaustauschermittel (3) ein mit Gas oder Gasgemisch mit Hilfe eines Brenners gespeistes Rohr umfassen, umfassend einen Mantel, der eine Ringkammer um diesen Brenner bildet, in dem das Gas oder das Wärmeaustauschgasgemisch zirkuliert und dann nach außen bezüglich dieser Kammer abgezogen wird.

6. Vorrichtung nach einem der Ansprüche 1 bis 5, bei dem wenigstens gewisse dieser Wärmeaustauschermittel (3) ein Rohr in Fingerhandschuhform umfassen, das einen gegen eines ihrer Enden geschlossenen Mantel und einen Innenmantel aufweist, der im wesentlichen koaxial zu diesem Aussenmantel ist, an seinen beiden Enden offen ist und mit Gas oder Wärmeaustauschgasgemisch über eines dieser Enden gespeist ist, wobei dieses Gas oder Wärmeaustauschgasgemisch abgezogen wird über das andere Ende in den freien Raum zwischen den beiden Mänteln.

7. Vorrichtung nach Anspruch 6, bei der wenigstens gewisse dieser Wärmeaustauschermittel (3) ein Rohr in Fingerhandschuhform umfassen, das mit Gas oder Gasgemisch mit Hilfe eines Brenners gespeist wird, der einen Mantel umfaßt, welcher eine Ringkammer um diesen Brenner formt, in welchem das Gas oder Wärmeaustauschgasgemisch, das aus dem Ringraum zwischen den beiden Mänteln des Rohrs in Fingerhandschuhform stammt, zirkuliert und dann aus dieser Kammer nach außen abgezogen wird, wobei der Außenmantel des Rohrs in Fingerhandschuhform und der Mantel die Ringkammer um den Brenner bilden und die im wesentlichen koaxial und vorzugsweise vom gleichen Innendurchmesser sind.

8. Vorrichtung nach Anspruch 6 oder 7, bei dem der Aussenmantel des Rohrs in Fingerhandschuhform und der die Ringkammer um den Brenner herum bildende Mantel aus ein und dem gleichen einzigen Rohr gebildet sind.

9. Vorrichtung nach einem der Ansprüche 6 bis 8, bei dem der Innenmantel des Fingerhandschuhrohres auf seiner radial außen liegenden Fläche Positionierungsrippen im Außenmantel des Fingerhandschuhrohres umfasst.

10. Vorrichtung nach einem der Ansprüche 6 bis 9, bei dem der Innenmantel des Fingerhandschuhrohres eine Vielzahl von Rohrstücken umfasst, die stirnseitig im wesentlichen axial ausgerichtet angeordnet und über Montagemittel zusammengefügt sind, die ein Ringorgan umfassen, das eine Hülle bildet, die koaxial die zu verbindenden Rohrstücke in Höhe der Verbindungszone umschließt und deren radial innen gelegene Fläche in der Mitte ihrer Länge einen Ringbund umfasst, der radial nach innen vorsteht und zwischen zwei zu verbindene Rohrstücke eingreift, wobei die Teile der Innenfläche, die zu beiden Seiten des Bundes angeordnet sind, allmählich gegen die Ende der Hülle aufweitend verlaufen.

11. Vorrichtung nach Anspruch 10, bei der die Hülle auf ihrer zylindrischen radialen Aussenfläche Zentrierungsrippen für den Innenmantel des Fingerhandschuhrohrs im Aussenmantel des Fingerhandschuhrohrs trägt.

12. Vorrichtung nach einem der Ansprüche 1 bis 11, bei der der Reaktor zusätzliche Wärmeaustauschermittel umfasst, die aus einem Rohr gebildet sind und einen mit Gas oder Gasgemisch nicht gespeisten Mantel umfassen, wobei diese Mittel so passive Wärmeübertragungsmittel bilden.

13. Vorrichtung nach einem der Ansprüche 1 bis 12, bei der der Reaktor (auf der Seite des ersten Endes) die erste Zone umfasst, in der wenigstens ein Teil und bevorzugt der grössere Teil der Wärmeaustauschermittel dieser Zone Heizmittel für den oder die in diesen Reaktor eingeführten Reaktionsteilnehmer bilden und die zweite Zone umfasst, in der wenigstens ein Teil und bevorzugt der grössere Teil der Wärmeaustauschermittel dieser zweiten Zone Mittel zur Kühlung oder zum indirekten Abschrecken der aus dieser ersten Zone stammenden Produkte bilden.

14. Vorrichtung nach Anspruch 13, bei der die Wärmeaustauschermittel für die erste mit Gas gespeiste Zone je mit Verbrennungsgasen bei erhöhter Temperatur gespeist werden, die aus einem Brenner stammen, der mit Brennstoff und mit die Verbrennung bewirkendem Mittel wie Luft gespeist sind und die mit Gas gespeisten Wärmeaustauschermittel der zweiten Zone je mit einem Gas bei geringerer Temperatur als dem der Verbrennungsgase, die diese erste Zone speisen, gespeist sind und bevorzugt durch Gase, die aus einem Brenner stammen, der mit einem die Verbrennung bewirkenden Mittel wie Luft ausschließlich gespeist wird.

15. Vorrichtung nach einem der Ansprüche 6 bis 14, bei der die Fingerhandschuhrohre Mäntel umfassen, bei denen das Verhältnis der Innenfläche (Sₑ) des geraden Querschnitts des den Außenmantel des Fingerhandschuhrohres bildenden Rohres zur Außenfläche (Sᵢ) des Querschnitts des den Innenmantel des Fingerhandschuhrohres bildenen Rohres gleich etwa 1,4:1 bis etwa 25:1 beträgt.
